# EUROPEAN PATENT SPECIFICATION

(11) **EP 1 068 861 B1**
(45) Date of publication and mention of the grant of the patent: **21.02.2007**
(21) Application number: 99113669.8
(22) Date of filing: 15.07.1999
(51) Int. Cl.: A61Q 5/08, A61Q 5/10, G01N 21/29, G01N 33/48, G01N 21/64, G01N 33/50

(54) **Method for measuring hair quality parameters**
Verfahren zur Messung von Qualitätsparametern von Haaren
Procédé de mesure de paramètres relatives à la qualité des cheveux

(43) Date of publication of application: 17.01.2001
(73) Proprietor: Amornsiripanitch, Somnuk, Pakkred, Nonthaburee 11120 (TH)
(72) Inventor: Amornsiripanitch, Somnuk, Pakkred, Nonthaburee 11120 (TH)
(74) Representative: HOFFMANN EITLE

(56) References cited:
- EP-A- 0 440 907
- US-A- 4 170 827
- US-A- 5 331 472
- PATENT ABSTRACTS OF JAPAN vol. 1997, no. 03, 31 March 1997 (1997-03-31) & JP 08 308814 A (ABE YASUHIKO), 26 November 1996 (1996-11-26)
- PATENT ABSTRACTS OF JAPAN vol. 018, no. 351 (C-1220), 4 July 1994 (1994-07-04) & JP 06 090932 A (SHISEIDO CO LTD;OTHERS: 01), 5 April 1994 (1994-04-05)
- PATENT ABSTRACTS OF JAPAN vol. 1995, no. 06, 31 July 1995 (1995-07-31) & JP 07 063534 A (KAWASAKI STEEL CORP), 10 March 1995 (1995-03-10)
- A. MATTE: ARCHIV FÜR KLINISCHE UND EXPERIMENTELLE DERMATOLOGIE, vol. 228, no. 1, 1967, pages 1-13, XP002121895

## Description

### Background of the invention

### Field of the Invention

The present invention relates to a method of measuring quality parameters of hair such as the diameter of hair shafts, the rate growth of hair shafts, and the total time in each phase of a hair growing cycle. The method can be carried out repeatedly to observe the dynamic changes of these parameters, and the method can also be used to study whether the change of these hair parameters are affected by foods, drugs, certain products, activities and habits.

### Description of Related Art

Hair problems and diseases, particularly thinning hairs and furs, are very common in both human and animals. Thin hairs and furs presumably are caused by specific changes of the hair growth cycle. If a high percentage of hairs or furs on the body part of interest is not growing out hair shafts, those hairs and furs are in the resting or catagen phase of the hair cycle. The resting hairs will fall out in the very near future, and at this stage the hairs or furs will not grow further if one shaves the area. Hair follicles left over after the shedding of hair shafts will not grow new hair shafts, and if the process takes a longer time, the number of hair shafts per unit area will decrease and cause empty areas at the spot. In another case new hairs can grow out immediately, but the new hairs are generating hair shafts with a significantly slow speed, and then the new hairs will not match the surrounding normal hairs in length, which will give the appearance of thinning hairs. The size of the hair shafts also determines the look of either thick or thin hairs since larger hair shaft will either reflect or absorb light, depending on the colour of the hair, better than hair with small diameter shafts. The ability of each hair follicle to generate more than one hair shaft at a time will also give a larger amount of hair in the area.

All the parameters regarding the generated hair shafts should be measured during the anagen or growing phase of the hair cycle. The steady growth rate of each hair shaft is also important to achieve the smooth silky hair and fur. Finally, the total duration of the growing phase will dictate when each hair will cease growing and return into the resting phase before it falls out to complete the whole cycle. Any factors possibly forcing the hair root to cease growing will shorten the duration of this growing phase and automatically increase the number of hairs getting into the resting phase.

If there were any versatile method for measuring the percentage of growing hair shafts and non-growing or non-performing hair shafts, calculating the approximate time new hair takes to grow out from the empty follicles, measuring the rate of generating hair shaft among all of the growing ones, counting the total number of hair density in the area, measuring the size of all the hair shafts in the observed unit field and finally monitoring all the changes of these parameters during the entire growing, resting and falling phase, the outcome of these data would be of advantage for many people. Physicians would be able to classify human hair problems more accurately and on a pathophysiological basis. Veterinarians would understand how to improve the nature of furs both in normal and ill animals. Food and drug industries could use the method to study many potential food supplements, herbal products and drugs possibly influencing each specific quality feature of hair through the whole cycle. Meticulously combining many factors with clear effects but with different roles would enhance the significant results of achieving good healthy hairs and furs. The method also could be used to identify the negative effects of other foods, products, drugs, activities and habits common to people and animals in daily life, and this information would help the related industries upgrade and improve the qualities of their products mentioned to benefit the consumers. Government could use the method to screen cosmetic hair care products and hair styling devices for safety and benefits of people. However, the fact is there are still no methods that have such capabilities.

Currently available methods can calculate the percentage of growing hair shafts versus non-growing portions, but the methods require the examined body part to be shaved and observed several days later. The methods cannot be used on unshaved area and do not lead to any conclusion on the duration of a hair growth cycle, which would be desirable, as stated in the previous paragraph. Furthermore, changes of hair shaft diameter and growing rate through time on the same hair shaft cannot be measured and recorded.

Although a hair density measuring method is also available with specific devices obtaining the photo or image for manual counting, the method cannot give information of how the hair density has changed.

To be precise, all available methods also cannot closely observe and record the dynamic changes of all the parameters throughout the life cycle.

### Summary of the invention

The invention provides a new method of measuring most of the parameters required to evaluate the complete quality of human and animal hairs in the observed area. The method comprises the features recited in claim 1. The following results can be obtained:
1. The exact percentage of non-growing hairs versus growing ones in the area of measuring.
2. The approximate gap time of an individual hair follicle between the point of hair shedding and the early sign of regenerating a young tip of a new hair shaft.
3. The rate of an individual hair shaft being produced per day at any point in time through its whole life cycle.
4. The diameter of each hair shaft correlating with the data in 3.
5. The amount of hair moving into and out of the resting phase each month.
6. The total growing duration of an individual hair starting from the early day until it transcends into the resting phase.
7. The total time of a hair staying in the non-growing phase before being shed out.
8. The budding activities of a follicle to give more than one hair shaft per follicle.
9. The dynamic changes of hair shaft parameters in the follicles mentioned in 8.

All the results can be recorded and retrieved for further statistic calculations and comparisons to see the effects of many unknown and known factors ranging from foods, drugs, hair care products, activities and habits on the health and quality of hair.

### Details of the invention

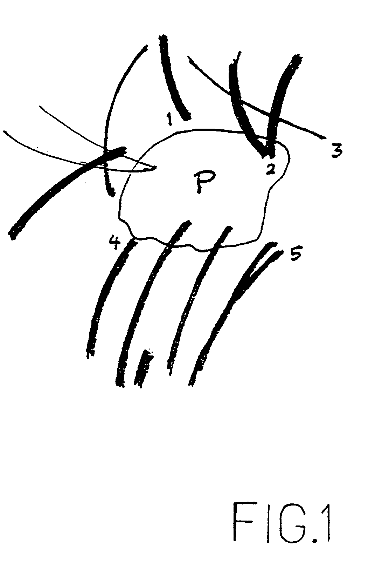

A specific portion of skin with hairs is selected, giving the priority to areas with natural pigmented lesions such as nevi, moles, lentigenes, seborrheic keratosis, or telangiectasia. These lesions can be easily located each time it is desired to reexam the changes of all the hair quality parameters. In addition, the small lesion (P) easily identified in the field of exam can help orientating the specific location of each hair follicle, as seen in Fig. 1. This simple technique will give the observer the good opportunity to see the specific changes of each identified hair shaft through time of hair measuring.

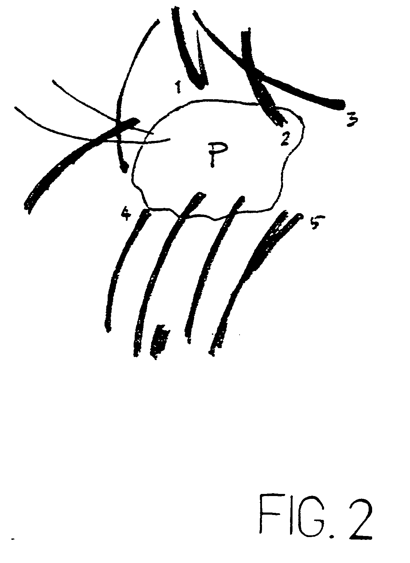

Without any preparation, the scalp with the natural marker as perfect indicator will provide the information of hair density, hair size and the rough estimation of whether an individual hair is growing or not by comparing the actual pictures of the spot over time as in Fig. 2. Follicle 1 shows the new hair tip generated out of the well-established hair shaft. Follicle 2 shows the disappearing of the previous hair shaft. Follicle 3 shows the change in hair shaft diameter.

The attempt to use this natural marker as a simple and good indicator for the mentioned advantages has never been observed and brought into practice. However, in the case there is no natural marker to work with, a photo at either the top or lateral view of scalp can be taken as a map to identify the approximate spots being examined. A few areas are chosen for examination since the more data is available, the more accurate the analysing step using basic statistic calculation will be.

The method according to the invention also includes a simple hair dying step. Several colours of hair dye creams and bleaching solutions easily found in supermarkets are used, with a small amount such as 0.3 ml per measured area. The selected dye with a specific colour (brown, blue, green, red etc.) permanently stains the hair shaft after about an hour of application (the exact time being previously calibrated as later detailed). The area then is washed with shampoo or simply wiped clean. The area is looked at through an electronic cameral device with enough magnifying power to observe the stained area. Successfully decolourising all the hair shafts at the point of hair shafts growing out of the skin completes this step (also it will help calibrating the exact time of the decolourising step depending on different brands of the dye and colours).

Twenty four to one hundred sixty eight hours (1-7 days) later the same area then is observed under the camera, and the image is captured under the observer's supervision. The angle of the camera will be adjusted until the new image has the similar orientation of all the hairs surrounding the marker as in previous images stored during the first examination.

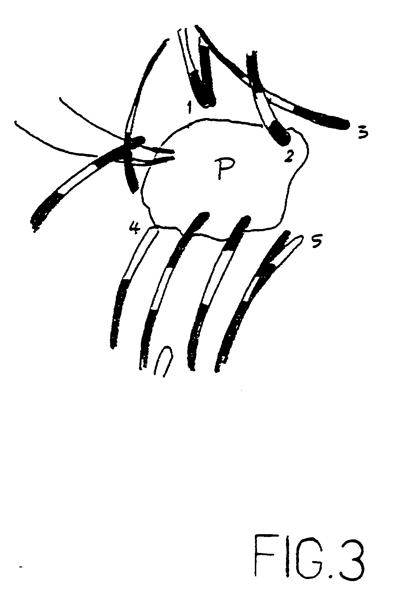

In Fig. 3, representing the new image, there are some hairs with natural hair colour growing out of the subject's skin with varieties of their length as seen in follicle number 1, 2 and 3. A number of them are still coloured with the selected dye as viewed during the end of the dying step (follicle number 4 and 5). From this data, a percentage of growing hairs and non-performing hairs can be recorded for future comparison.

To calculate this growing rate of each hair shaft, the length of each hair shaft with normal hair colour must be measured one by one. The data is then subjected to calculation by using the magnifying power of the recording camera and the total amount of time between the time point of completing the dying step and the time point of having the dyed area measured again. This can be done manually or automatically by having a prepared table or mathematics program containing the result of the known equation that can find the actual growth rate. The growth rate is equal to the measured length (millimetre or inch) obtained from the image divided by the product of magnifying factor and time gap (recorded in hours or days). Entering the obtained data into the normal statistical calculating practice can derive the value of the maximum, the minimum, the mode, the median of the whole area growth rate with standard deviation and variance. The value will be compared with the other values obtained either from the same area but at a different time or from a different area to reach the conclusion whether there is significant changes of hair growth rate.

Observing the dynamic changes of individual non-growing hairs until each of them falls out will give the period of time between the beginning of the resting phase and the day they shed out. During this period, a manual technique or device is included to gently pull those hairs to specify the point of time the hair root gets loose and is ready to fall out. Once the follicles have emptied the hair threads, the spots are continuously observed to see when the tip of new hairs is starting to grow out of the follicles. The time it takes until the tip of hair is generated is recorded and represents the total time of this segment of the hair cycle.

During this step, there is another benefit of this invention which can be explained as follows. After the gathering data of non-growing hair percentage, the observer can weekly observe the progressing change of this percentage by seeing the decrease amounts of previous stained colour hair shafts at the junction of those hair shafts and the skin. The decrease number of stained hairs mentioned in the previous sentence should equal the amount of hairs falling out of the examined area. However, the left over stained hairs are not representing the total amount of hairs resting in the area since some of the unstained shafts may already cease to grow at any time during this step of viewing. The viewing only allows to determine how many resting hairs from the previous observation are either left over or pushed out before the new examination will be carried out. This information will help interpreting the result from the new round of staining test to obtain information about how many non-growing hairs are the new ones recently moving into the resting phase.

By means of this type of observation the dynamic changes of the hairs moving into and out of the resting phase can then be measured quite accurately. The amount of total hairs moving into the resting phase at a specific range of time will be important for further analysis. Monitoring the lone percentage of the non-growing hairs in the area at any point in time can then be specified in more detail by using this step of approach. This technical step can provide the following results:
1. dynamic change of the hairs moving into and out of the resting phase,
2. a correct answer of whether, if the amount of hairs in resting phase has increased, the amount of hairs moving into the resting phase is more or the amount of hairs moving out of the resting phase is less, and
3. a hologram demonstrating the amount of hair moving or shedding out on the x axis and how frequently the area has shed out the hair on the y axis.

The total benefits of this simple dying technique have not been available before, and there were no technique which could perform this simple method and achieve these benefits. The invention does not only allow to find the approximate time of the hair cycle starting from the ceasing point of hair growth to the point of falling and the time spent in generating the new tip of hair out of the empty follicle, but the invention also has the capability to measure the total growing time starting from the date of new hair tipping out of its under skin follicle to the day it ceases to grow, which is another important phase of the whole cycle of an individual hair.

Choosing several colours of hair dye and recording the sequence of the colours and the exact date may also be included in this step of the invention. Staining the hair shaft as mentioned earlier, among these hair follicles, a few new hairs just entering the growing phase will tip out of the new reform hair follicles. The colour will stain both the shafts of old hairs and the tip of new hairs. The colour stained at the tip will tell the observer when it was stained. Each colour used will have its own date of record. Consequently, the similar area is subjected to the dying step monthly, every two months, quarterly or biannually, depending on the protocol and relying on the status of growth rate obtained from the first measuring and depending on how wide the segment of hair shaft is which is being stained each time.

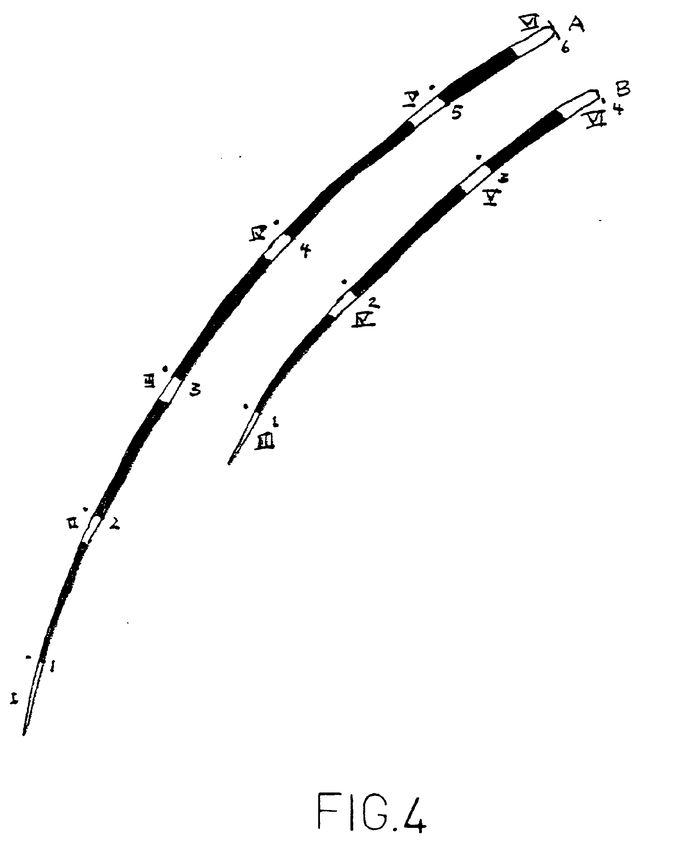

However, each new dying step has to use the planned sequence of colours prepared for the whole method, and each dye should be at least different enough from the natural colour hair shaft. With a successful process, as in Fig. 4, the segment of the colour dyed hair shaft will significantly differ from the natural colour band. By repeating the step of staining and waiting periodically as planned, any fallen hair will have the zebra bands of artificial colours switched with the natural colour on its whole length. The artificial colours will be arranged on the whole range of the hair thread specifically as planned (I, II, III, IV, V and VI). All the hairs with their tips stained are collected, and each segment of natural colour from point 1 to point 2, point 2 to point 3, point 3 to the end of hair root is measured. The measuring will go on from the first segment till the end of the hair root, no matter how many bands there are on each hair specimen. Hair A has six segments stained with colour I to colour VI while hair. B has only three segments stained with colour III to colour VI. The distance between point 1 and point 2 timed with the rate of growth of the segment will give the duration of time it spends to generate it. Simple adding all the data obtained from each segment calculating will then give the total approximated duration of growth of that specific hair.

In a different approach, once the hair with stained tip cease growing, cutting the hair for similar measuring will give more specific time of growing phase, not including the total time of resting. Naturally, in the same area, each new hair grows out at a different time. However, hairs with a similar tip colour are assumed to grow out at the same time. The colour at the tip of each hair then logically represents each hair group growing out form the same area but at a different time frame. Further comparing the duration of growth of each hair group will give the valuable insight whether there is any improvement of hair quality with regard to the total duration of growth.

The inventive method of producing the unique hair specimens described for measuring the total duration of growth will help specifying the information obtained from the previous step mentioned above. No similar technique has ever been mentioned to provide the opportunity to study the quality of hair in this dimension.

To capture all mentioned images, an electronic camera such as A 71-50N Scarlar CCD scanner (Scalar Corporation 3-28-6 Yoyogi Shibuya-ku Tokyo Japan) can be used to obtained the still electronic images. The still images can then be transferred through either Asymetrix Digital Video Producer Capture version 5.0 (wwww.asymetrix.com) or Aver (AverMedia Technologies, Inc) to the Microsoft window bitmap paint program. Both window 3.11 version and window 95 version can be used to store the images. The images can then be archived and retrieved for measuring to reach the data. A statistical analysing program such as a SPSS program (SPSS.co) can be used for the calculation.

## Claims

1. A method for measuring the quality of hairs, the method comprising the following steps:
a. identifying a selected skin or scalp area, preferably an area comprising a pigmented marker, for which a hair quality measurement is desired,
b. taking a first optical record of this area to document its initial status,
c. bleaching or dying the hair shafts in the selected area,
d. taking a 2^{nd} optical record of the selected area to confirm the completely coloured status,
e. taking a final optical record of the selected area after 1 to 7 days, and
f. processing data obtained from the first and final optical record to determine hair parameter changes.

2. The method according to claim 1, wherein said hair parameters are the hair density, the amounts of growing and non-growing hairs, the hair shaft diameter, and/or the number of hair shafts growing out of the scalp in the selected area.

3. The method according to claim 1 or 2; wherein after step f., the following further step is carried out:
g. counting and measuring coloured and non-coloured hair shafts and tips for calculating hair growth.

4. The method according to any one of the preceding claims, wherein steps a. to f. or a. to g., respectively, are repeated at least once at intervals of a month, two months, or half a year.

5. The method according to claim 4, wherein alternating bands of coloured segments and natural colour segments produced by the method are used for measuring hair quality parameters.

6. The method according to any one of the preceding claims, wherein after step g., non-growing hair is picked to specify the point of time the hair root gets loose and is ready to fall out, and the date of picking is recorded.

7. The method according to any one of the preceding claims, whether the selected area is identified by using a device for mechanically and/or electronically marking the area.

8. The method according to any one of the preceding claims, wherein the dying step uses fluorescence dyes.

9. The method according to any one of the preceding claims, wherein a device with the capability of automatically matching an old image with a newly taken image is used to stored all the optical records.

10. The method according any one of the preceding claims, wherein the obtained optical records are stored and archived to allow them to be retrieved for further statistic calculations.

11. The method according to any one of the preceding claims, wherein the results of steps a. to g. are further used to determine the effects of any type of foods, drugs, products, devices, activities and habits on the hair quality.

## Patentansprüche

1. Verfahren zum Messen der Qualität von Haaren, wobei das Verfahren die folgenden Schritte umfasst:
a. Identifizieren eines ausgewählten Haut- oder Kopfhautbereichs, vorzugsweise eines Bereichs mit einer pigmentierten Markierung, für den eine Haar-Qualitätsmessung gewünscht wird,
b. Aufnehmen einer ersten optischen Aufnahme dieses Bereichs, um seinen anfänglichen Status zu dokumentieren,
c. Bleichen oder Färben der Haar-Schäfte in dem ausgewählten Bereich,
d. Aufnehmen einer zweiten optischen Aufnahme des ausgewählten Bereichs, um den vollständig kolorierten Zustand zu bestätigen,
e. Aufnehmen einer optischen Schluss-Aufnahme des ausgewählten Bereichs nach 1 bis 7 Tagen, und
f. Verarbeiten der von der ersten optischen Aufnahme und der optischen Schluss-Aufnahme erhaltenen Daten, um Haar-Parameteränderungen zu bestimmen.

2. Verfahren nach Anspruch 1, bei dem die Haar-Parameter die Haardichte, die Menge von wachsenden und nicht wachsenden Haaren, der Haar-Schaftdurchmesser, und/oder die Anzahl von Haar-Schäften sind, die aus der Kopfhaut in dem ausgewählten Bereich heraus wachsen.

3. Verfahren nach Anspruch 1 oder 2, bei dem nach dem Schritt f. der folgende weitere Schritt durchgeführt wird:
g. Zählen und Messen von kolorierten und nicht kolorierten Haar-Schäften und Spitzen zur Berechnung des Haarwachstums.

4. Verfahren nach einem der vorhergehenden Ansprüche, bei dem die Schritte a. bis f., beziehungsweise a. bis g., zumindest einmal in Intervallen von einem Monat, zwei Monaten, oder einem halben Jahr, wiederholt werden.

5. Verfahren nach Anspruch 4, bei dem abwechselnde Bänder von kolorierten Segmenten und Segmenten mit natürlicher Farbe, die durch das Verfahren erzeugt werden, zum Messen von Haar-Qualitätsparametern verwendet werden.

6. Verfahren nach einem der vorhergehenden Ansprüche, bei dem nach Schritt g. nicht wachsendes Haar gezupft wird, um den Zeitpunkt zu spezifizieren, an dem die Haarwurzel lose wird und bereit ist auszufallen, und wobei das Datum des Zupfens aufgenommen wird.

7. Verfahren nach einem der vorhergehenden Ansprüche, bei dem der ausgewählte Bereich durch die Verwendung einer Vorrichtung zum mechanischen und/oder elektronischen Markieren des Bereichs identifiziert wird.

8. Verfahren nach einem der vorhergehenden Ansprüche, bei dem der Färbe-Schritt Fluoreszenz-Färbemittel verwendet.

9. Verfahren nach einem der vorhergehenden Ansprüche, bei dem eine Vorrichtung mit der Fähigkeit, automatisch ein altes Bild mit einem neu aufgenommenen Bild zu vergleichen, verwendet wird, um sämtliche der optischen Aufnahmen zu speichern.

10. Verfahren nach einem der vorhergehenden Ansprüche, bei dem die erhaltenen optischen Aufnahmen gespeichert und archiviert werden, um zuzulassen, dass sie für weitere statische Berechnungen wieder abgerufen werden.

11. Verfahren nach einem der vorhergehenden Ansprüche, bei dem die Ergebnisse der Schritte a. bis g. ferner verwendet werden, um die Wirkungen von jeglicher Art von Nahrungsmitteln, Arzneimitteln, Produkten, Vorrichtungen, Aktivitäten und Gewohnheiten, auf die Haar-Qualität zu bestimmen.

## Revendications

1. Procédé de mesure de la qualité des cheveux, le procédé comprenant les étapes suivantes :
a. identification d'une zone choisie de peau ou de cuir chevelu, de préférence une zone comprenant un marqueur pigmenté, pour lequel une mesure de la qualité du cheveu est souhaitée,
b. prise d'un premier enregistrement optique de cette zone pour décrire son état de départ,
c. décoloration ou coloration des tiges capillaires dans la zone choisie,
d. prise d'un second enregistrement optique de la zone choisie pour confirmer l'état entièrement coloré,
e. prise d'un enregistrement optique final de la zone choisie, après 1 à 7 jour(s), et
f. traitement des données obtenues à partir du premier et du dernier enregistrements optiques pour déterminer les changements de paramètres du cheveu.

2. Procédé selon la revendication 1, dans lequel lesdits paramètres du cheveu sont la densité des cheveux, les quantités de cheveux qui poussent et qui ne poussent pas, le diamètre de la tige capillaire, et/ou le nombre de tiges capillaires croissant en dehors du cuir chevelu dans la zone choisie.

3. Procédé selon la revendication 1 ou 2, dans lequel après l'étape f., l'étape supplémentaire suivante est conduite :
g. décompte et mesure des tiges et des extrémités capillaires colorées et non colorées pour le calcul de la croissance du cheveu.

4. Procédé selon l'une quelconque des revendications précédentes, dans lequel les étapes a. jusqu'à f. ou a. jusqu'à g., respectivement, sont répétées au moins une fois à des intervalles d'un mois, deux mois, ou de la moitié d'une année.

5. Procédé selon la revendication 4, dans lequel des bandes alternées de segments colorés et de segments de couleur naturelle produits par le procédé sont utilisées pour mesurer les paramètres de qualité du cheveu.

6. Procédé selon l'une quelconque des revendications précédentes, dans lequel après l'étape g., le cheveu qui ne pousse pas est repéré pour spécifier le moment où la racine capillaire devient lâche et est prête à tomber, et la date du repérage est enregistrée.

7. Procédé selon l'une quelconque des revendications précédentes, que la zone choisie soit identifiée en utilisant un dispositif de marquage mécanique et/ou électronique de la zone.

8. Procédé selon l'une quelconque des revendications précédentes, dans lequel l'étape de coloration utilise des colorants fluorescents.

9. Procédé selon l'une quelconque des revendications précédentes, dans lequel un dispositif avec la capacité de faire correspondre automatiquement une ancienne image avec une image nouvellement prise est utilisé pour stocker tous les enregistrements optiques.

10. Procédé selon l'une quelconque des revendications précédentes, dans lequel les enregistrements optiques obtenus sont stockés et archivés pour leur permettre d'être récupérés pour des calculs statistiques ultérieurs.

11. Procédé selon l'une quelconque des revendications précédentes, dans lequel les résultats des étapes a. jusqu'à g. sont en outre utilisés pour déterminer les effets de n'importe quel type d'aliments, substances médicamenteuses, produits, dispositifs, activités et habitudes sur la qualité du cheveu.
